(19) Europäisches Patentamt
European Patent Office
Office européen des brevets

(11) EP 1 757 935 A1

(12) EUROPEAN PATENT APPLICATION
published in accordance with Art. 158(3) EPC

(43) Date of publication:
**28.02.2007 Bulletin 2007/09**

(21) Application number: **05737304.5**

(22) Date of filing: **25.04.2005**

(51) Int Cl.:
***G01N 33/53*** (2006.01) ***C12Q 1/68*** (2006.01)
***G01N 37/00*** (2006.01) *C12N 15/09* (2006.01)

(86) International application number:
**PCT/JP2005/008374**

(87) International publication number:
**WO 2005/103695 (03.11.2005 Gazette 2005/44)**

(84) Designated Contracting States:
**AT BE BG CH CY CZ DE DK EE ES FI FR GB GR HU IE IS IT LI LT LU MC NL PL PT RO SE SI SK TR**

(30) Priority: **23.04.2004 JP 2004128940**

(71) Applicants:
- **Matsushita Electric Industries Co., Ltd.**
  **Kadoma-shi,**
  **Osaka 571-8501 (JP)**
- **Microtec Co. Ltd.**
  **Funabashi-shi, Chiba 274-0074 (JP)**

(72) Inventors:
- **MAEDA, Mizuo**
  **Tokyo 140-0014 (JP)**
- **AKIMOTO, Katsuhiko,**
  **c/o Microtec Co.,Ltd.**
  **Funabashi-shi,**
  **Chiba 274-0074 (JP)**
- **HORI, Junichi,**
  **c/o Matsushita Electric Ind.Co.,Ltd**
  **Chuo-ku,**
  **Osaka-shi, Osaka 540-6319 (JP)**
- **MURAYAMA, Ryusuke,**
  **Matsushita Electric Ind.Co.,Ltd**
  **Chuo-ku,**
  **Osaka-shi, Osaka 540-6319 (JP)**
- **TABATA, Jinpei,**
  **c/o Matsushita Electric Ind.Co.Ltd**
  **Chuo-ku,**
  **Osaka-shi, Osaka 540-6319 (JP)**
- **BANDO, Katsuhiko,**
  **Matsushita Electric Ind.Co.,Ltd**
  **Chuo-ku,**
  **Osaka-shi, Osaka 540-6319 (JP)**

(74) Representative: **Pautex Schneider, Nicole Véronique**
**Novagraaf International SA**
**25, Avenue du Pailly**
**1220 Les Avanchets - Geneva (CH)**

# (54) GENE DETECTION METHOD AND GENE DETECTION APPARATUS

(57) There are provided a gene detection method and an apparatus thereof, for detecting a gene having a specific sequence in a specimen with high sensitivity. The gene detection method comprises a gene sample conformation step of conforming a gene sample by denaturing a gene to be detected in the specimen into a single strand; an immobilization step of immobilizing a single-stranded nucleic acid probe having a base sequence that is complementary to the gene sequence to be detected, onto an electrode; a hybridization step of adding the single-stranded gene sample to the electrode on which the single-stranded nucleic acid probe is immobilized, thereby forming a double-stranded nucleic acid in which the nucleic acid probe and the gene sample are hybridized; an intercalator addition step of adding an intercalator that is electrochemically active and is covalently bonded to the double-stranded nucleic acid by light irradiation, to the electrode on which the double-stranded nucleic acid is formed; a light irradiation step of covalently bonding the double-stranded nucleic acid and the intercalator by performing light irradiation; a washing step of removing the intercalator that is unreacted with the double-stranded nucleic acid; and a detection step of detecting the intercalator that is covalently bonded to the double-stranded nucleic acid, by electrochemical measurement after the washing step.

EP 1 757 935 A1

Fig.3

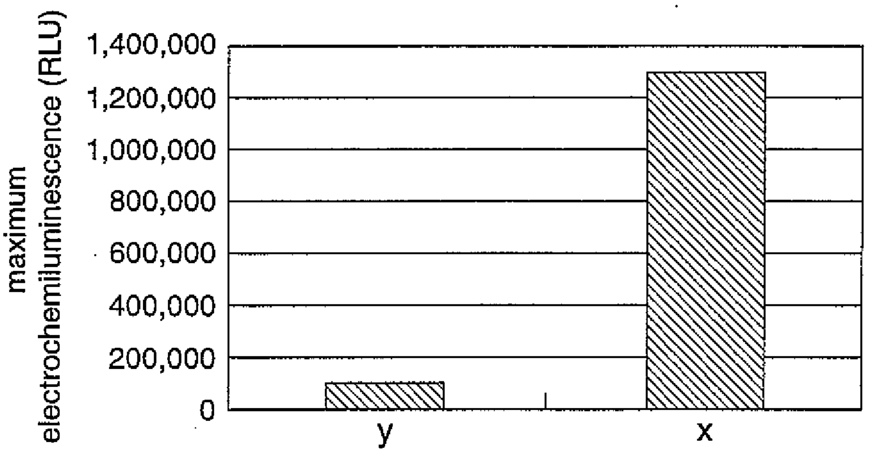
maximum electrochemiluminescence (RLU)
1,400,000
1,200,000
1,000,000
800,000
600,000
400,000
200,000
0
y
x

SEQUENCE LISTING

<110> Matsushita Electric Industrial Co., Ltd.

<120> Gene Detection Method and Gene Detection Apparatus

<130> P38717-P0

<150> JP 2004-128940
<151> 2004-04-23

<160> 3

<210> 1
<211> 40
<212> DNA
<213> Homo sapiens

<220>
<223> 5'-terminal phosphate group modified by thiol group

<400> 1
cccccctggat ccagatatgc aataattttc ccactatcat 40

<210> 2
<211> 40
<212> DNA
<213> Homo sapiens

<400> 2
atgatagtgg gaaaattatt gcatatctgg atccaggggg 40

<210> 3
<211> 40
<212> DNA
<213> Artificial Sequence

<400> 3
aaaaaaaaaa aaaaaaaaaa aaaaaaaaaa aaaaaaaaaa 40

## Description

### TECHNICAL FIELD

The present invention relates to a gene detection method and an apparatus thereof, which can detect a target gene having a specific gene sequence of a sample by an electrochemiluminescence signal of an intercalator.

### BACKGROUND ART

In a conventional method for electrochemically detecting a specific gene sequence, a single-stranded nucleic acid probe having a base sequence that is complementary to a target gene to be detected is immobilized on an electrode surface, and the nucleic acid probe and the target gene sample that is denatured to a single strand are hybridized, and thereafter, an intercalator which is electrochemically active and specifically binds to the double-stranded nucleic acid comprising the nucleic acid probe and the target gene sample is added to a reaction system for the nucleic acid probe and the gene sample. Then, a voltage is applied to the reaction system to which the intercalator is added to perform electrochemical measurement, and the intercalator bonded to the double-stranded nucleic acid is detected, whereby the nucleic acid probe that is hybridized with the target gene sample is detected to confirm existence of the target gene (for example, refer to Patent Publication No.2573443 (Patent Document 1).

The intercalator indicates a substance that recognizes the double-stranded nucleic acid and specifically binds to the double-stranded nucleic acid. The intercalator has a tabular intercalation base such as phenyl in a molecule, and binds to the double-stranded nucleic acid by that the intercalation base is intercalated between a base pair and a base pair of the double-stranded nucleic acid. This binding of the intercalator and the double-stranded nucleic acid is a binding due to electrostatic interaction or hydrophobic interaction, and it is a binding due to equilibrium reaction in which intercalation of the intercalator between the base pairs of the double-stranded nucleic acid, and separation of the intercalator from between the base pairs are repeated at a constant speed.

Among intercalators, there is a substance that causes electrically reversible oxidation-reduction reaction. By using such intercalator that causes electrochemically reversible oxidation-reduction reaction, it is possible to detect existence of the intercalator that binds to the double-stranded nucleic acid by measuring the electrochemical change. This electrochemical change can be detected by current or luminescence that occurs during oxidation-reduction.

That is, in the conventional gene detection method, it is important that the intercalator specifically binds to only the double-stranded nucleic acid, and that the amount of the intercalator bonded to the double-stranded nucleic acid is accurately detected.

However, the intercalator used for the conventional gene detection is nonspecifically adsorbed to the single-stranded nucleic acid probe and to the electrode surface onto which the nucleic acid probe is immobilized. The nonspecifically adsorbed intercalator causes background noise when detecting the amount of the intercalator that binds to the double-stranded nucleic acid, leading to reduction in the detection sensitivity.

In order to solve this problem, in the conventional method, after the intercalator is added to the reaction system for the nucleic acid probe and the gene sample, it is necessary to perform a washing process for removing the intercalator that is nonspecifically adsorbed to the single-stranded nucleic acid probe and the electrode surface (for example, refer to Patent Publication No.3233851 (Patent Document 2).

### DISCLOSURE OF THE INVENTION

### PROBLEMS TO BE SOLVED BY THE INVENTION

However, as described above, since the intercalator and the double-stranded nucleic acid are bonded to each other by electrostatic interaction or hydrophobic interaction, the bonding force between them is weak, and the intercalator bonded to the double-stranded nucleic acid is undesirably dissociated during the above-mentioned washing process, whereby the detection sensitivity is reduced.

On the other hand, when the washing process is carried out so as to prevent the dissociation of the intercalator bonded to the double-stranded nucleic acid, removal of the intercalator that is nonspecifically adsorbed to the single-stranded nucleic acid probe and the electrode surface becomes insufficient, and the background noise cannot be sufficiently removed, leading to reduction in the detection sensitivity.

Furthermore, since the bonding reaction between the intercalator and the double-stranded nucleic acid is equilibrium reaction, the ratio of the intercalator intercalated between the base pairs of the double-stranded nucleic acid is low, and therefore, the detection sensitivity is low even when the above-mentioned background noise does not occur.

The present invention is made to solve the above-mentioned problems and has for its object to provide a gene detection method and an apparatus thereof, which can detect a gene in a specimen with high sensitivity.

MEASURES TO SOLVE THE PROBLEMS

In order to solve the conventional problems, a gene detection method for detecting a gene having a specific sequence in a specimen, comprises a gene sample conformation step of conforming a gene sample by denaturing a gene to be detected in the specimen into a single strand; an immobilization step of immobilizing a single-stranded nucleic acid probe having a base sequence that is complementary to the gene sequence to be detected, onto an electrode; a hybridization step of adding the single-stranded gene sample to the electrode on which the single-stranded nucleic acid probe is immobilized, thereby forming a double-stranded nucleic acid in which the nucleic acid probe and the gene sample are hybridized; an intercalator addition step of adding an intercalator that is electrochemically active and is covalently bonded to the double-stranded nucleic acid by light irradiation, to the electrode on which the double-stranded nucleic acid is formed; a light irradiation step of covalently bonding the double-stranded nucleic acid and the intercalator by performing light irradiation; a washing step of removing the intercalator that is unreacted with the double-stranded nucleic acid; and a detection step of detecting the intercalator that is covalently bonded to the double-stranded nucleic acid, by electrochemical measurement after the washing step.
Therefore, the double-stranded nucleic acid obtained by hybridizing the gene sample and the nucleic acid probe can be irreversibly and firmly bonded to the intercalator, whereby the gene in the specimen can be detected with high sensitivity. Further, during the washing step, while the intercalator bonded to the double-stranded nucleic acid is not dissociated from the nucleic acid, the intercalator that is nonspecifically adsorbed to the single-stranded nucleic acid probe or the surface of the electrode can be removed, whereby the gene in the specimen can be detected with high sensitivity.
Further, in the gene detection method of the present invention, the electrochemical measurement comprises applying a voltage to the electrode, and measuring an amount of electrochemiluminescence due to the intercalator that is covalently bonded to the double-stranded nucleic acid.
Therefore, the double-stranded nucleic acid immobilized onto the electrode can be detected with high sensitivity, and consequently, the gene sample that is hybridized with the nucleic acid probe can be detected with high sensitivity.
Further, in the gene detection method of the present invention, the intercalator comprises a compound having a double-stranded nucleic acid bonding site that is specifically intercalated into the double-stranded nucleic acid, and forms covalent bonding with the double-stranded nucleic acid by light irradiation, an electrochemical active site having electrochemical activity, and a connection site that connects the double-stranded nucleic acid bonding site with the electrochemical active site.
Therefore, the double-stranded nucleic acid and the intercalator can be irreversibly and firmly bonded, and consequently, the ratio of the intercalator to be intercalated into the double-stranded nucleic acid is increased, whereby the target gene sample can be detected with high sensitivity.
Further, in the gene detection method of the present invention, the double-stranded nucleic acid bonding site is an intercalator having photosensitivity.
Therefore, the double-stranded nucleic acid and the intercalator can be irreversibly and firmly bonded by light irradiation.
Further, in the gene detection method of the present invention, the intercalator having photosensitivity is furocoumarin derivative.
Further, in the gene detection method of the present invention, the furocoumarin derivative is psoralen derivative.
Further, in the gene detection method of the present invention, the compound having oxidation-reduction property is a compound indicating electrochemiluminescence.
Therefore, when a voltage is applied to the electrode, the intercalator that is bonded to the double-stranded nucleic acid immobilized onto the electrode performs oxidization-reduction and simultaneously emits light, whereby the target gene sample can be detected by measuring the amount of electrochemiluminescence.
Further, in the gene detection method of the present invention, the compound indicating electrochemiluminescence is a metal complex having a heterocyclic system compound as a ligand, which is selected from a group consisting of rubrene, anthracene, coronene, pyrene, fluoranthene, chrysene, phenanthrene, perylene, binaphthyl, octatetraene.
Further, in the gene detection method of the present invention, the metal complex having a heterocyclic system compound as a ligand is a metal complex having a pyridine site.
Further, in the gene detection method of the present invention, the metal complex having a pyridine site as a ligand is a metal bipyridine complex or a metal phenanthroline complex.
Further, in the gene detection method of the present invention, a center metal of said metal complex having a heterocyclic system compound as a ligand is ruthenium or osnium.
Therefore, when a voltage is applied to the electrode, more favorable amount of electrochemiluminescence can be obtained, whereby the gene sample can be detected with higher sensitivity.
Furthermore, a gene detection apparatus for detecting a gene having a specific sequence in a specimen, comprises an electrode on which a single-stranded nucleic acid probe having a base sequence that is complementary

to the gene sequence to be detected is immobilized; a hybridization tank for forming a double-stranded nucleic acid by hybridizing the nucleic acid probe immobilized on the electrode, with a gene sample that is obtained by denaturing the gene to be detected in the specimen into a single strand; an intercalator addition tank for adding an intercalator that is electrochemically active and is covalently bonded to the double-stranded nucleic acid by light irradiation, onto the electrode, and performing light irradiation to covalently bond the double-stranded nucleic acid with the intercalator; a washing tank for removing, with a washing solution, the intercalator that is unreacted with the double-stranded nucleic acid; a detection tank for detecting the intercalator covalently bonded to the double-stranded nucleic acid, by electrochemical measurement; and an electrode moving unit for moving the electrode into the respective tanks in order of the hybridization tank, the intercalator addition tank, the washing tank, and the detection tank.

Therefore, it is possible to provide a gene detection apparatus that can detect the gene in the specimen with high sensitivity by detecting, with electrochemical measurement, the intercalator that is irreversibly and firmly bonded to the double-stranded nucleic acid obtained by hybridizing the gene sample and the nucleic acid probe. Further, since the intercalator that is nonspecifically adsorbed to the single-stranded nucleic acid or the electrode surface can be removed in the washing tank without dissociating the intercalator bonded to the double-stranded nucleic acid, the gene in the specimen can be detected with high sensitivity.

Furthermore, a gene detection apparatus for detecting a gene having a specific sequence in a specimen, comprises an electrode on which a single-stranded nucleic acid probe having a base sequence that is complementary to the gene sequence to be detected is immobilized; a gene sample conformation unit for conforming a gene sample by denaturing the gene to be detected into a single strand; an intercalator tank for holding an intercalator that is electrochemically active and is covalently bonded to the double-stranded nucleic acid by light irradiation; a washing solution tank for holding a washing solution that removes the intercalator unreacted with the double-stranded nucleic acid; an electrolyte tank for holding an electrolyte for detecting the intercalator covalently bonded to the double-stranded nucleic acid; and a processing tank for forming a double-stranded nucleic acid by the nucleic acid probe immobilized onto the electrode and the gene sample, covalently bonding the double-stranded nucleic acid and the intercalator by light irradiation, washing the intercalator that is unreacted with the double-stranded nucleic acid by the washing solution, and detecting the intercalator covalently bonded to the double-stranded nucleic acid by electrochemical measurement, said processing tank being connected to the gene sample conformation unit, the intercalator tank, the washing solution tank, and the electrolyte tank.

Therefore, it is possible to provide a gene detection apparatus that can detect the gene in the specimen with high sensitivity by detecting, with electrochemical measurement, the intercalator that is irreversibly and firmly bonded to the double-stranded nucleic acid obtained by hybridizing the gene sample and the nucleic acid probe. Further, since the gene detection apparatus is provided with only one processing tank, the size of the apparatus can be reduced. Moreover, when the intercalator that is unreacted with the double-stranded nucleic acid is removed by the washing solution, it is possible to remove the intercalator that is nonspecifically adsorbed to the single-stranded nucleic probe or the electrode surface, without dissociating the intercalator bonded to the double-stranded nucleic acid, thereby providing a compact gene detection apparatus that can detect the gene in the specimen with high sensitivity.

## EFFECTS OF THE INVENTION

According to the gene detection method and apparatus of the present invention, when detecting a gene having a specific sequence, an intercalator that is electrochemically active and is covalently bonded to the double-stranded nucleic acid by light irradiation is used. Therefore, the double-stranded nucleic acid and the intercalator can be covalently bonded by light irradiation, whereby the double-stranded nucleic acid and the intercalator can be bonded irreversibly and firmly, and consequently, the gene sample can be detected with high sensitivity.

Further, according to the gene detection method and apparatus, after the double-stranded nucleic acid and the intercalator are covalently bonded by light irradiation, washing is carried out to remove the intercalator that is nonspecifically adsorbed to the single-stranded nucleic acid and the electrode surface. Therefore, while the intercalator bonded to the double-stranded nucleic acid is not dissociated by a washing solution, the intercalator that is nonspecifically adsorbed to the single-stranded nucleic acid probe and the electrode surface can be removed, whereby the gene in the specimen can be detected with high sensitivity.

## BRIEF DESCRIPTION OF THE DRAWINGS

Figure 1 is a diagram illustrating a construction of a gene detection apparatus according to a first embodiment of the present invention.

Figure 2 is a diagram illustrating another construction of a gene detection apparatus according to a second embodiment of the present invention.

Figure 3 is a diagram illustrating the maximum amounts of electrochemiluminescence detected on a gold electrode x on which double-stranded nucleic acid is formed and on a gold electrode y on which no double-stranded nucleic acid is formed, which electrodes are fabricated in an process of a first example of the present invention.

DESCRIPTION OF THE REFERENCE NUMERALS

| | |
|---|---|
| 1 ... | gene sample conformation unit |
| 2 ... | electrode |
| 3 ... | hybridization tank |
| 4,10 ... | temperature controller |
| 5,25 ... | electrode moving unit |
| 5a ... | electrode holding arm |
| 5b ... | arm driving unit |
| 6 ... | gene sample washing solution tank |
| 7 ... | gene sample washing tank |
| 8 ... | intercalator tank |
| 9 ... | intercalator reaction tank |
| 11 ... | UV lamp |
| 12 ... | intercalator washing solution tank |
| 13 ... | intercalator washing tank |
| 14 ... | electrolyte tank |
| 15 ... | detection tank |
| 16 ... | photo multiplier |
| 17 ... | potentiostat |
| 18 ... | controller |
| 23 ... | processing tank |
| 27 ... | waste solution tank |
| 100,200 ... | gene detection apparatus |

BEST MODE TO EXECUTE THE INVENTION

Hereinafter, a gene detection method according to the present invention will be described in detail.

In the following embodiments of the invention, a gene sample is obtained as follows. That is, from an arbitrary sample including, for example, blood, white blood cell, blood serum, urine, feces, semen, saliva, cultured cell, tissue cell such as cells of various organs, and gene, a double-stranded nucleic acid is released by disrupting the cell in the sample, and then the double-stranded nucleic acid is dissociated into a single-stranded nucleic acid by thermal treatment or alkali treatment. Further, the gene sample in the embodiments of the invention may be a nucleic acid segment that is cut off by a restriction enzyme and purified by such as separation using electrophoresis.

[Embodiment 1]

Hereinafter, a gene detection method according to a first embodiment will be described.

Initially, a gene sample to be a test object is formed. This gene sample is obtained by, as described above, a cell in an arbitrary sample is disrupted to release a double-stranded nucleic acid and then the double-stranded nucleic acid is denatured into a single-stranded nucleic acid by thermal treatment or alkali treatment.

At this time, the cell in the sample can be disrupted by an ordinary method, such as vibration or application of a physical effect such as ultrasonic wave from the outside. Further, it is also possible to release the nucleic acid from the cell by using a nucleic acid extraction solution (for example, a surface-active agent such as SDS, Triton-X, or Tween-20, or a solution including such as saponin, EDTA, or protease).

Next, a single-stranded nucleic acid probe having a base sequence that is complementary to the gene sequence to be detected is formed.

As for this nucleic acid probe, it is possible to use a nucleic acid that is obtained by cutting off a nucleic acid extracted from a biologic sample, with a restriction enzyme, and purifying the nucleic acid by electrophoresis separation or the like, or a single-stranded nucleic acid obtained by chemical synthesis. In the case of using the nucleic acid extracted from the biologic sample, it is preferable to dissociate the same into a single-stranded nucleic acid by thermal treatment

or alkali treatment.

Thereafter, the nucleic acid probe obtained as described above is immobilized onto an electrode.

Any electrode may be used in the present invention so long as it can be used as an electrode. For example, there may be used a noble metal electrode such as gold, platinum, platinum black, palladium, or rhodium, a carbon electrode such as graphite, glassy carbon, pyrolytic graphite, carbon paste, or carbon fiber, an oxide electrode such as titanic oxide, tin oxide, manganese oxide, or lead oxide, and a semiconductor electrode such as Si, Ge, ZnO, CdS, TiO, or GaAs. These electrodes may be covered with a conductive polymer, thereby to prepare a more stable probe immobilized electrode.

As a method for immobilizing the nucleic acid probe to the electrode, a well-know method is adopted. For example, when the electrode comprises gold, thiol group is introduced to a 5'- or 3'- terminal (preferably, 5'- terminal) of the nucleic acid probe to be immobilized, and the nucleic acid probe is immobilized to the metal electrode via covalent bond between gold and sulfur. A method for introducing the thiol group to the nucleic acid probe is described in "M. Maeda et al., Chem. Lett., 1805~1808 (1994)", and "B.A. Connolly, Nucleic Acids Res., 13, 4484 (1985)".

That is, the nucleic acid probe having the thiol group obtained by the above-mentioned method is dropped onto the gold electrode, and the gold electrode is left for a few hours under a low temperature, whereby the nucleic acid probe is immobilized onto the electrode, resulting in a nucleic acid probe.

Another method is as follows. For example, when the electrode comprises glassy carbon, initially the glassy carbon is oxidized with potassium permanganate to introduce carboxylic acid group onto the electrode surface, whereby the nucleic acid probe is immobilized onto the surface of the glassy carbon electrode by amide binding. A substantial method for immobilizing the nucleic acid probe onto the glassy carbon electrode is described in "K. M. Millan et al., Analytical Chemistry, 65, 2317~2323 (1993) ".

Next, the electrode onto which the nucleic acid probe is immobilized is brought into contact with a solution including the gene sample. Thereby, the immobilized nucleic acid probe and the gene sample having a sequence that is complementary to the nucleic acid probe are hybridized, thereby generating a double-stranded nucleic acid on the electrode. Since the method for hybridizing the nucleic acid probe and the gene sample is well known, a description thereof will be omitted.

After the double-stranded nucleic acid is formed on the electrode, an intercalator is added to the electrode on which the double-stranded nucleic acid is formed so that the intercalator is intercalated into and reacted with the double-stranded nucleic acid. The intercalator may be added to the sample before the formation of the double-stranded nucleic acid, that is, before the hybridization.

Thereafter, the double-stranded nucleic acid to which the intercalator is added is irradiated with light to make covalent bonding between the double-stranded nucleic acid and the intercalator.

Hereinafter, a description will be given of the intercalator according to the first embodiment.

The present invention adopts, as an intercalator, a substance that can be specifically intercalated into the double-stranded nucleic acid, and covalently bonded to the double-stranded nucleic acid by light irradiation.

Since the intercalator is firmly and irreversibly bonded to the double-stranded nucleic acid, the intercalator bonded to the double-stranded nucleic acid is not dissociated from the double-stranded nucleic acid during the following washing process, and only the unreacted intercalator can be removed during the washing process.

Further, the present invention adopts an electrochemically active substance as an intercalator.

Therefore, it is possible to detect existence of the double-stranded nucleic acid with high sensitivity, by an electrochemical signal that is derived from the intercalator specifically bonded to the double-stranded nucleic acid.

An intercalator that satisfies the above-mentioned two characteristics is a compound having a double-stranded nucleic acid binding site (I) that can be specifically intercalated into the double-stranded nucleic acid and covalently bonded to the double-stranded nucleic acid by light irradiation, an electrochemical active site (F) that has electrochemical activity, and a connecting site (L) that connects the double-stranded nucleic acid binding site (I) with the electrochemical active site (F).

For example, the intercalator adopted in the present invention can be expressed by the following general formula (1).

$$F - L - I \qquad ...(1)$$

wherein F is electrochemical active group, L is connecting group, and I is double-stranded nucleic acid intercalation group having a site that is linked with the double-stranded nucleic acid by light irradiation.

As a substance that can be used as the double-stranded nucleic acid intercalation group I shown in the general formula (1), there is a photosensitive intercalator which is a substance that can be specifically intercalated into the double-stranded nucleic acid and covalently bonded to the double-stranded nucleic acid by light irradiation.

For example, furocoumarin derivative is adopted as a photosensitive intercalator, and particularly, psoralen derivative is preferable. When the psoralen derivative is intercalated into the double-stranded nucleic acid, it causes noncovalent interaction with the double-stranded nucleic acid. Further, when it is irradiated with a long wavelength ultraviolet ray (300~400nm), the psoralen derivative portion intercalated into the double-stranded nucleic acid is covalently bonded to the double-stranded nucleic acid firmly and irreversibly, resulting in stable covalent bonding.

Accordingly, in the washing process described later, when the single-stranded nucleic acid probe that is immobilized onto the electrode surface where no double-stranded nucleic acid is formed and the intercalator that is nonspecifically adsorbed to the electrode surface are removed, the intercalator bonded to the double-stranded nucleic acid is prevented from falling off, and therefore, the unreacted single-stranded nucleic acid probe and the nonspecifically adsorbed intercalator can be reliably removed by performing strong washing during the washing process.

As specific examples of the psoralen derivative, there are psoralen, methoxypsoralen, and trimethylpsoralen.

Next, a substance that can be used as the electrochemical active group F shown in the general formula (1) may be any substance so long as it is electrochemically detectable. For example, there is a compound having oxidation-reduction property, which is detectable by measuring oxidation-reduction current that occurs during reversible oxidation-reduction reaction.

As examples of the compound having oxidation-reduction property, there are ferrocene, catecholamine, a metal complex having a heterocyclic system compound as a ligand, rubrene, anthracene, coronene, pyrene, fluoranthene, chrysene, phenanthrene, perylene, binaphthyl, octatetraene, and viologen.

Further, among the compounds having oxidation-reduction property (i.e., a metal complex having a heterocyclic system compound as a ligand, rubrene, anthracene, coronene, pyrene, fluoranthene, chrysene, phenanthrene, perylene, binaphthyl, and octatetraene), some of them occur electrochemiluminescence during oxidation-reduction reaction, and the substance can be detected by measuring the electrochemiluminescence.

As examples of the metal complex having a heterocyclic system compound as a ligand, there are heterocyclic system compounds including oxygen or nitrogen, for example, metal complexes having a pyridine site or a pyran site as ligands, and particularly, a metal complex having a pyridine site as a ligand is preferable. As examples of the metal complex having a pyridine site as a ligand, there are a metal bipyridine complex and a metal phenanthroline complex.

Further, as examples of a center metal of the metal complex having a heterocyclic system compound as a ligand, there are ruthenium, osnium, zinc, cobalt, platinum, chrome, molybdenum, tungsten, technetium, rhenium, rhodium, iridium, palladium, copper, indium, lanthanum, praseodymium, neodymium, and samarium. Particularly, a complex having ruthenium or osnium as a center metal has favorable electrochemiluminescent characteristics. As examples of a material having favorable electrochemiluminescent characteristics, there are ruthenium bipyridine complex, ruthenium phenanthroline complex, osnium bipyridine complex, and osnium phenanthroline complex.

In the general formula (1), as for a group that can be used as the connecting group L, its linker sequence is not particularly restricted so long as the connecting group L can bind the electrochemical active group F and the double-stranded nucleic acid intercalation group I. For example, alkyl group, - O- group, -CO- group, -NH- group, phosphate group, or a combination of these groups can be used.

The intercalator as described above is added before or after the hybridization of the gene sample and the nucleic acid probe, and then the double-stranded nucleic acid in which the nucleic acid probe and the gene sample are hybridized is covalently bonded to the intercalator by light irradiation, and thereafter, electrode washing process is carried out.

During the washing process, unreacted single-stranded nucleic acid probe that is immobilized to the electrode surface, and the intercalator that is nonspecifically adsorbed to the electrode surface are removed.

As the result, only the intercalator that is specifically covalently bonded to the double-stranded nucleic acid remains in the hybridized double-stranded nucleic acid, and existence of the double-stranded nucleic acid can be detected with high sensitivity by measuring an electrochemical signal that is derived from the intercalator.

Although it depends on the type of an intercalator to be added, the electrochemical signal derived from the intercalator can be measured by a measurement system comprising a potentiostat, a function generator, and the like, when an intercalator that generates oxidation-reduction current is used. On the other hand, the electrochemical signal can be measured by a photo multiplier or the like, when an intercalator that generates electrochemiluminescence is used.

Hereinafter, a gene detection apparatus according to the present invention will be described with reference to figure 1. Figure 1 is a diagram illustrating the construction of a gene detection apparatus according to the first embodiment. In figure 1, a gene detection apparatus 100 has five kinds of tanks, i.e., a hybridization tank 3, a gene sample washing tank 7, an intercalator reaction tank 9, an intercalator washing tank 13, and a detection tank 15. Reference numeral 1 denotes a gene sample conformation unit for conforming a gene sample to be a test object, from a cell in a sample, and numeral 2 denotes an electrode to which a single-stranded nucleic acid probe having a sequence that is complementary to the gene sample to be detected is immobilized. Reference numeral 5 denotes an electrode moving unit which comprises an electrode holding arm 5a for holding the electrode 2 and an arm driving part 5b for driving the electrode holding arm 5a, and moves the electrode 2 successively into the above-mentioned five tanks 3, 7, 9, 13 and 15. For example, the

electrode moving unit 5 is realized by a transport arm or a loader such as a belt conveyer.

Reference numeral 6 denotes a gene sample washing solution tank which holds a solution for washing an unreacted gene sample at the surface of the electrode 2, numeral 8 denotes an intercalator tank which holds an intercalator, numeral 12 denotes an intercalator washing solution tank which holds a solution for washing the unreacted intercalator at the surface of the electrode 2, and numeral 14 denotes an electrolytic solution tank which holds an electrolytic solution.

The gene sample conformation unit 1 is connected to the hybridization tank 3, the gene sample washing solution tank 6 is connected to the gene sample washing tank 7, the intercalator tank 8 is connected to the intercalator reaction tank 9, the intercalator washing solution tank 12 is connected to the intercalator washing tank 13, and the electrolytic solution tank 14 is connected to the detection tank 15. Further, the intercalator reaction tank 9 is provided with a UV lamp 11 which irradiates the electrode 2 with light. The detection tank 15 is provided with a potentiostat 17 which applies voltage to the electrode 2, a photo multiplier 16 which measures electrochemiluminescence of the electrode 2, and a control unit 18 which controls the voltage applied to the electrode 2, and analyzes the measurement result obtained from the photo multiplier 16.

Further, the hybridization tank 3 and the intercalator reaction tank 9 are fitted to temperature controllers 4 and 10, respectively.

Next, the operation of the apparatus 100 will be described.

Initially, a target cell including a gene to be detected is inputted to the gene sample conformation unit 1, thereby forming a sample solution including a gene sample in which a target gene is denatured into a single strand. Then, the sample solution including the gene sample is sent to the hybridization tank 3, and the sample solution is dropped onto the electrode 2 on which the nucleic acid probe is immobilized, which electrode 2 is set in the hybridization tank 3. At this time, the temperature in the hybridization tank 3 should be controlled appropriately by the temperature controller 4.

At the surface of the electrode 2 onto which the sample solution is dropped, the nucleic acid probe immobilized to the electrode 2 and the gene sample having a sequence complementary to the nucleic acid probe are hybridized, thereby forming a double-stranded nucleic acid.

After the reaction is completed, the electrode 2 is moved into the gene sample washing tank 7 by the electrode moving unit 5.

In the gene sample washing tank 7, the washing solution held in the gene sample washing solution tank 6 is dropped onto the electrode 2, and the unreacted gene sample at the surface of the electrode 2 is removed with the washing solution.

After the washing is completed, the electrode 2 is moved into the intercalator reaction tank 9 by the electrode moving unit 5.

In the intercalator reaction tank 9, the intercalator held in the intercalator tank 8 is dropped onto the electrode 2, whereby the intercalator is intercalated into and reacted with the double-stranded nucleic acid. At this time, the temperature in the intercalator reaction tank 9 should be controlled appropriately by the temperature controller 4.

Thereafter, in the intercalator reaction tank 9, the electrode 2 onto which the intercalator is dropped is irradiated with light using the UV lamp 11, whereby the intercalator and the double-stranded nucleic acid are covalently bonded.

After the reaction is ended, the electrode 2 is moved into the intercalator washing tank 13 by the electrode moving unit 5.

In the intercalator washing tank 13, the washing solution held in the intercalator washing solution tank 12 is dropped onto the electrode 2, and the unreacted intercalator at the surface of the electrode 2 is removed with the washing solution.

After the washing is ended, the electrode 2 is moved into the detection tank 15 by the electrode moving unit 5.

In the detection tank 15, the electrode 2 is connected to the potentiostat 17, and the electrolytic solution held in the electrolytic solution tank 14 is dropped onto the electrode 2.

Under the control of the controller 18, the potentiostat 17 applies voltage to the electrode 2 to generate electrochemiluminescence. Then, the photo multiplier 16 measures the electrochemiluminescence, and the measurement result is inputted to the controller 18 to be analyzed.

As described above, according to the first embodiment, as an intercalator to be intercalated into a double-stranded nucleic acid that is obtained by hybridizing a gene as a target of detection and a nucleic acid probe having a sequence that is complementary to the target gene, a substance that is electrochemically active and is covalently bonded to the double-stranded nucleic acid by light irradiation is employed. Therefore, the double-stranded nucleic acid and the intercalator are covalently bonded by light irradiation, whereby the double-stranded nucleic acid and the intercalator can be bonded irreversibly and firmly. Consequently, highly-precise measurement result can be obtained, and a target gene having a specific sequence can be detected with high sensitivity. Further, according to the first embodiment, since the intercalator and the double-stranded nucleic acid are covalently bonded, the intercalator bonded to the double-stranded nucleic acid is not dissociated even when the unreacted intercalator at the surface of the electrode 2 is removed, while the intercalator that is nonspecifically adsorbed to the single-stranded nucleic acid probe or the surface of the electrode

can be removed. Therefore, the gene in the sample can be detected with high sensitivity.

While in the first embodiment there are provided, as washing tanks, the gene sample washing tank 7 for washing the unreacted gene sample after the hybridization process, and the intercalator washing tank 13 for washing the unreacted intercalator after the intercalator addition process, a single washing tank may be shared by these washing processes, or only the unreacted intercalator washing process may be performed without performing the unreacted gene sample washing process.

Further, while in the above-mentioned gene detection apparatus 100 a tank is provided for each process to be performed on the electrode 2, all the processes may be performed with a single tank.

[Embodiment 2]

While in the first embodiment the plural tanks for performing the plural treatments on the electrode 2 are provided to perform the respective treatments with the different tanks, in this second embodiment, the respective treatments to the electrode 2 are performed with a single tank.

Figure 2 is a diagram illustrating the construction of a gene detection apparatus according to the second embodiment. With reference to figure 2, the gene detection apparatus 200 has a processing tank 23 for performing the respective treatments onto the electrode 2. Reference numeral 25 denotes an electrode moving unit for moving the electrode in the horizontal direction in the processing tank 23. For example, a mechanism for horizontally moving a stage may be used. Reference numeral 27 denotes a waste solution tank into which the solution collected in the processing tank 23 is discharged.

The processing tank 23 contains the electrode 2 onto which the nucleic acid probe is immobilized, and the electrode moving unit 25 for moving the electrode 2 in the horizontal direction, and further, the gene sample conformation unit 1, the gene sample washing solution tank 6, the intercalator tank 8, the intercalator washing solution tank 12, the electrolytic solution tank 14, and the waste solution tank 27 are connected to the processing tank 23.

Further, the processing tank 23 is provided with the UV lamp 11 for irradiating the electrode 2 with light, and the photo multiplier 16 for measuring electrochemiluminescence of the electrode 2, and further, the processing tank 23 is fitted to the temperature controller 4.

Furthermore, the apparatus 200 is provided with the potentiostat 17 for applying voltage to the electrode 2, and the controller 18 for controlling the voltage applied to the electrode 2, and analyzing the measurement result obtained from the photo multiplier 16.

Next, the operation of the apparatus 200 will be described.

Initially, a test target cell including a gene to be detected is inputted to the gene sample conformation unit 1, thereby conforming a sample solution including a gene sample in which the target gene is denatured into a single strand. Then, the sample solution including the gene sample is sent to the processing tank 23, and the sample solution is dropped onto the electrode 2 on which the nucleic acid probe is immobilized, which electrode 2 is set in the processing tank 23. At this time, the temperature in the processing tank 23 should be controlled appropriately by the temperature controller 4.

At the surface of the electrode 2 onto which the sample solution is dropped, the nucleic acid probe immobilized to the electrode 2 and the gene sample having a sequence complementary to the nucleic acid probe are hybridized, thereby forming a double-stranded nucleic acid.

After the reaction is ended, the washing solution held in the gene sample washing solution tank 6 is dropped onto the electrode 2, and the unreacted gene sample at the surface of the electrode 2 is removed with the washing solution. At this time, the electrode moving unit 25 moves the electrode 2 in the horizontal direction to a predetermined position so that the washing solution sent from the gene sample washing solution tank 6 is appropriately dropped onto the electrode 2. Further, the washing solution dropped onto the electrode 2 is collected into the waste solution tank 27.

After the washing is ended, the intercalator held in the intercalator tank 8 is dropped onto the electrode 2, whereby the intercalator is intercalated into and reacted with the double-stranded nucleic acid. At this time, the electrode moving unit 25 moves the electrode 2 in the horizontal direction to a predetermined position so that the intercalator sent from the intercalator tank 8 is appropriately dropped onto the electrode 2. Further, the temperature in the processing tank 23 is controlled appropriately by the temperature controller 4.

After the reaction is ended, the washing solution held in the intercalator washing solution tank 12 is dropped onto the electrode 2, and the unreacted intercalator at the surface of the electrode 2 is removed with the washing solution. At this time, the electrode moving unit 25 moves the electrode 2 in the horizontal direction to a predetermined position so that the washing solution sent from the intercalator washing solution tank 12 is appropriately dropped onto the electrode 2. Further, the washing solution dropped on the electrode 2 is collected into the waste solution tank 27.

After the washing is ended, the electrode 2 is connected to the potentiostat 17, and the electrolytic solution held in the electrolytic solution tank 14 is dropped onto the electrode 2. At this time, the electrode moving unit 25 moves the electrode 2 in the horizontal direction to a predetermined position so that the electrolytic solution sent from the

electrolytic solution tank 14 is appropriately dropped onto the electrode 2.

Under the control of the controller 18, the potentiostat 17 applies voltage to the electrode 2 to generate electrochemiluminescence. Then, the photo multiplier 16 measures the electrochemiluminescence, and the measurement result is inputted to the controller 18 to be analyzed.

As described above, according to the second embodiment, as an intercalator to be intercalated into a double-stranded nucleic acid that is obtained by hybridizing a gene as a target of detection and a nucleic acid probe having a sequence that is complementary to the target gene, a substance that is electrochemically active and is covalently bonded to the double-stranded nucleic acid by light irradiation is employed. Therefore, the double-stranded nucleic acid and the intercalator are covalently bonded by light irradiation, whereby the double-stranded nucleic acid and the intercalator can be bonded irreversibly and firmly. Consequently, highly precise measurement result can be obtained, and a target gene having a specific sequence can be detected with high sensitivity. Further, according to the second embodiment, since the intercalator and the double-stranded nucleic acid are covalently bonded, the intercalator bonded to the double-stranded nucleic acid is not dissociated even when the unreacted intercalator at the surface of the electrode 2 is removed, while the intercalator that is nonspecifically adsorbed to the single-stranded nucleic acid probe or the surface of the electrode can be removed. Therefore, the gene in the sample can be detected with high sensitivity.

While in this second embodiment there are provided the gene sample washing solution tank 6 for washing the unreated gene sample after the hybridization process, and the intercalator washing solution tank 12 for washing the unreacted intercalator after the intercalator addition process, the processing tank 23 may be provided with a single washing solution tank, and the same washing solution may be used for both the unreacted gene sample washing process after the hybridization process, and the unreacted intercalator washing process after the light irradiation process. Alternatively, between the two washing processes, only the intercalator washing process may be carried out without performing the gene sample washing process.

EXAMPLE 1

Hereinafter, a practical example of the present invention will be described, but the present invention is not restricted thereto.

(1) Immobilization of Nucleic Acid Probe onto Metal Electrode surface

A gold electrode is prepared by depositing gold (200nm) with titan (10nm) as a base layer, on a glass substrate, by using a sputtering unit (SH-350 produced by U1VAC, Inc.), and then forming an electrode pattern in a photolithography process. The electrode surface is washed for one minute with piranha solution (hydrogen peroxide : concentrated sulfuric acid = 1 : 3), and rinsed with pure water, and then dried by nitrogen blow.

As a nucleic acid probe, there is employed 40-base oligodeoxynucleotide (produced by TAKARA BIO INC.) which has a sequence of CCCCCTGGAT CCAGATATGC AATAATTTTC CCACTATCAT positioned in the 629-668th from the 5'-terminal of a gene sequence of human-derived Cytochrome P-450, and is modified with thiol group via phosphate group at the 5'-terminal. Then, the nucleic acid probe is dissolved into 10mM of PBS (sodium phosphate buffer solution of pH7.4) to adjust it to 100$\mu$M.

Thus adjusted nucleic acid probe solution is dropped onto the gold electrode, and left for four hours at room temperature under saturated humidity, whereby the thiol group and the gold are bonded to immobilize the nucleic acid probe onto the gold electrode.

(2) Hybridization

As a gene sample, employed is oligodeoxynucleotide (produced by TAKARA BIO INC.) having a sequence of ATGATAGTGG GAAAATTATT GCATATCTGG ATCCAGGGGG from the 5'-terminal, which is complementary to the nucleic acid probe. The gene sample is dissolved into a hybridization solution in which 10mM of PBS and 2XSSC are mixed to adjust it to 20$\mu$M.

The adjusted hybridization solution in which the gene sample is dissolved is dropped onto the gold electrode on which the nucleic acid probe is immobilized, and the hybridization solution and the nucleic acid probe are reacted for four hours in a tank that is kept at constant temperature of 40°C, thereby forming double-stranded nucleic acid. Thus, a gold electrode x on which the double-stranded nucleic acid is formed is obtained.

Further, in this example, a gold electrode y on which no double-stranded nucleic acid is formed is prepared as a target for comparison. The gold electrode y having no double-stranded nucleic acid is obtained by using a gene sample having a sequence that is not complementary to the above-mentioned nucleic acid probe (hereinafter referred to as comparison gene sample), and subjecting the gene sample to the same processing as that performed for the gold electrode x on which double-stranded nucleic acid is formed. In this example, 40mer Poly-A (produced by TAKARA BIO

INC.) having a sequence of AAAAAAAAAA AAAAAAAAAA AAAAAAAAAA AAAAAAAAAA is adopted as the comparison gene sample.

(3) Addition of Intercalator

A psoralen-modified ruthenium complex expressed by the following chemical formula is used as an intercalator.

(Chemical Formula 1)

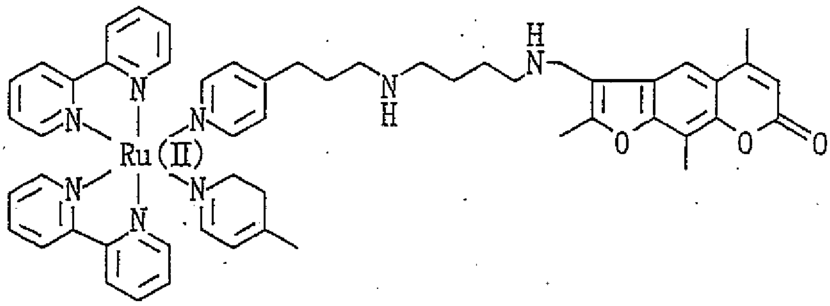

Synthesis of the psoralen-modified ruthenium complex is performed in the following procedure.

Initially, 4'-chloromethyl-4,5,8-trimethylpsoralen (0.5g, 1.81mmol) is dissolved in sodium hydroxide disolution dimethylformamide (dry), and 1,4-diaminobutane (0.32g, 3.63mmol) is dropped while agitating the solution at 160°C to promote reaction for twelve hours. After the solvent is distilled away, a crude product is purified by silica gel chromatography, thereby obtaining a product A (yield 40%).

Next, 2.50g ($1.35\times10^{-2}$mol) of 4,4'-dimethyl-2,2' bipyridine that is dissolved in 60.0mL of THF is injected into a container of nitrogen atmosphere, and thereafter, 16.9mL ($2.70\times10^{-2}$mol) of lithium diisopropylamide 2M solution is dropped, and the solution is agitated for thirty minutes while cooling the same. On the other hand, 7.61g ($4.05\times10^{-2}$mol) of 1.2-dibromoethane and 10mL of THF are added into a container that is similarly dried in nitrogen gas stream, and the solution is agitated while cooling the same.

The solution in which the 4,4'-dimethyl-2,2' bipyridine dissolved in the THF is reacted with the lithium diisopropylamide 2M solution is slowly dropped into the container that contains the 1.2-dibromoethane and the THF, and reaction is promoted for 2.5 hours. This reaction solution is neutralized with 2N of hydrochloric acid to distil the THF away, and then extracted by chloroform, and further, the crude product obtained by distilling the solvent away is purified by silica gel column, thereby obtaining a product B (yield 47%).

Then, the product A (0.50g, 1.52mmol) and the product B (0.49g, 1.68mmol) are dissolved into sodium hydroxide dissolution dimethylformamide (dry), and the solution is agitated for eighteen hours at 160°C. This agitated solvent is distilled away, and then the crude product is purified by silica gel chromatography, thereby obtaining a product C (yield 38%).

Further, ruthenium chloride (III) (2.98g, 0.01mol) and 2,2'-bipyridine (3.44g, 0.022mol) are refluxed for six hours in dimethylformamide (80.0mL), and then the solvent is distilled away. Thereafter, acetone is added, and a black sediment that is obtained by cooling the solution overnight is extracted. Then, 170mL of ethanol aqueous solution (ethanol : water = 1 : 1) is added, and the solution is heated to reflux for one hour. After filtration, 20g of lithium chloride is added, and ethanol is distilled away, and further, the resultant is cooled overnight. The deposited black substance is extracted by suction filtration, thereby obtaining a product D (yield 68.2%).

Then, the product C (0.30g, 0.56mmol) and the product D (0.32g, 0.66mmol) are dissolved in dimethylformamide, and the solution is refluxed for six hours. After the reaction, distilled water is added to a black-violet substance that is obtained by distilling the solvent away to dissolve the substance, and the unreacted complex is removed by filtration, and thereafter, the solvent is distilled away.

The crude product thus obtained is purified by silica gel chromatography, thereby obtaining psoralen-modified ruthenium complex (yield 68%). Table 1 shows the results of proton NMR ($^1$H-NMR) of the psoralen-modified ruthenium complex that is obtained as described above.

(Table 1)

$^1$H-NMR (300MHz, DMSOd-6)

σ :

| | |
|---|---|
| 1.4~1.8 | (6H, m) |
| 2.4~2.6 | (12H, m) |
| 2.74 | (2H, t) |
| 3.8~3.1 | (6H, m) |
| 4.31 | (2H, s) |
| 6.32 | (1H, s) |
| 7.38 | (2H, d) |
| 7.54 | (7H, m) |
| 7.77 | (4H, m) |
| 8.16 | (4H, t) |
| 8.70 | (2H, d) |
| 8.88 | (4H, d) |

The psoralen-modified ruthenium complex thus obtained is adjusted to 2 μM in 10mM of PBS.

The adjusted solution is applied to the gold electrode x on which the double-stranded nucleic acid is formed and to the gold electrode y on which no double-stranded nucleic acid is formed, respectively, and dark reaction is promoted for thirty minutes in a refrigerator at 4°C.

(4) Covalent Bonding between Double-Stranded Nucleic Acid and Intercalator

After thirty minutes, the respective gold electrodes x and y are irradiated with ultraviolet ray having a wavelength of 365nm (5mW/cm$^2$) for ten minutes using a UV cross linker (UVPCL-1000L type produced by FUNAKOSHI CO.,Ltd.), whereby the psoralen and the double-stranded nucleic acid are covalently bonded. After the covalent bonding, the gold electrodes x and y are respectively vibrated and washed for ten minutes in 10mM of PBS, thereby to remove unreacted Ru complex.

(5) Electrochemical Measurement

After the above-mentioned processes, an electrolytic solution in which 0.1M of PBS and 0.1M of triethylamine are mixed is dropped onto the gold electrode x and the gold electrode y on which no double-stranded nucleic acid is formed, respectively.

Thereafter, voltage is applied to the respective gold electrodes x and y, and intercalator-derived electrochemiluminescence which occurs at this time is measured.

The electrochemical measurement is carried out for one second by voltage scanning from 0V to 1.3V. The measurement of the amount of electrochemiluminescence is carried out using a photoelectron multiplier (H7360-01 produced by Hamamatsu Photonics K.K.), and a maximum amount of luminescence is measured.

Figure 3 shows the result obtained in the electrochemical measurement according to the example 1. As is evident from figure 3, the amount of luminescence on the gold electrode x on which the double-stranded nucleic acid is formed is significantly larger than the amount of luminescence on the gold electrode y on which no double-stranded nucleic acid is formed, and therefore, it is discovered that detection of the double-stranded nucleic acid can be performed with high sensitivity by using the intercalator according to the example 1.

APPLICABILITY IN INDUSTRY

A gene detection method according to the present invention can detects a gene having a specific sequence with high sensitivity, and it is applicable to gene examination, infection examination, genome-based drag discovery, and the like.

## Claims

**1.** A gene detection method for detecting a gene having a specific sequence in a specimen, said method comprising:

a gene sample conformation step of conforming a gene sample by denaturing a gene to be detected in the specimen into a single strand;
an immobilization step of immobilizing a single-stranded nucleic acid probe having a base sequence that is complementary to the gene sequence to be detected, onto an electrode;

a hybridization step of adding the single-stranded gene sample to the electrode on which the single-stranded nucleic acid probe is immobilized, thereby forming a double-stranded nucleic acid in which the nucleic acid probe and the gene sample are hybridized;
an intercalator addition step of adding an intercalator that is electrochemically active and is covalently bonded to the double-stranded nucleic acid by light irradiation, to the electrode on which the double-stranded nucleic acid is formed;
a light irradiation step of covalently bonding the double-stranded nucleic acid and the intercalator by performing light irradiation;
a washing step of removing the intercalator that is unreacted with the double-stranded nucleic acid; and
a detection step of detecting the intercalator that is covalently bonded to the double-stranded nucleic acid, by electrochemical measurement after the washing step.

**2.** A gene detection method as defined in Claim 1 wherein said electrochemical measurement comprises applying a voltage to the electrode, and measuring an amount of electrochemiluminescence due to the intercalator that is covalently bonded to the double-stranded nucleic acid.

**3.** A gene detection method as defined in Claim 1 wherein
said intercalator comprises a compound having
a double-stranded nucleic acid bonding site that is specifically intercalated into the double-stranded nucleic acid, and forms covalent bonding with the double-stranded nucleic acid by light irradiation,
an electrochemical active site having electrochemical activity, and
a connection site that connects the double-stranded nucleic acid bonding site with the electrochemical active site.

**4.** A gene detection method as defined in Claim 3 wherein said double-stranded nucleic acid bonding site is an intercalator having photosensitivity.

**5.** A gene detection method as defined in Claim 4 wherein said intercalator having photosensitivity is furocoumarin derivative.

**6.** A gene detection method as defined in Claim 5 wherein said furocoumarin derivative is psoralen derivative.

**7.** A gene detection method as defined in Claim 3 wherein said electrochemical active site is a compound having oxidation-reduction property.

**8.** A gene detection method as defined in Claim 7 wherein said compound having oxidation-reduction property is a compound indicating electrochemiluminescence.

**9.** A gene detection method as defined in Claim 8 wherein said compound indicating electrochemiluminescence is a metal complex having a heterocyclic system compound as a ligand, which is selected from a group consisting of rubrene, anthracene, coronene, pyrene, fluoranthene, chrysene, phenanthrene, perylene, binaphthyl, octatetraene.

**10.** A gene detection method as defined in Claim 9 wherein said metal complex having a heterocyclic system compound as a ligand is a metal complex having a pyridine site.

**11.** A gene detection method as defined in Claim 10 wherein said metal complex having a pyridine site as a ligand is a metal bipyridine complex or a metal phenanthroline complex.

**12.** A gene detection method as defined in Claim 9 wherein a center metal of said metal complex having a heterocyclic system compound as a ligand is ruthenium or osnium.

**13.** A gene detection apparatus for detecting a gene having a specific sequence in a specimen, said apparatus comprising:

an electrode on which a single-stranded nucleic acid probe having a base sequence that is complementary to the gene sequence to be detected is immobilized;
a hybridization tank for forming a double-stranded nucleic acid by hybridizing the nucleic acid probe immobilized on the electrode, with a gene sample that is obtained by denaturing the gene to be detected in the specimen into a single strand;

an intercalator addition tank for adding an intercalator that is electrochemically active and is covalently bonded to the double-stranded nucleic acid by light irradiation, onto the electrode, and performing light irradiation to covalently bond the double-stranded nucleic acid with the intercalator;
a washing tank for removing, with a washing solution, the intercalator that is unreacted with the double-stranded nucleic acid;
a detection tank for detecting the intercalator covalently bonded to the double-stranded nucleic acid, by electrochemical measurement; and
an electrode moving unit for moving the electrode into the respective tanks in order of the hybridization tank, the intercalator addition tank, the washing tank, and the detection tank.

**14.** A gene detection apparatus for detecting a gene having a specific sequence in a specimen, said apparatus comprising:

an electrode on which a single-stranded nucleic acid probe having a base sequence that is complementary to the gene sequence to be detected is immobilized;
a gene sample conformation unit for conforming a gene sample by denaturing the gene to be detected into a single strand;
an intercalator tank for holding an intercalator that is electrochemically active and is covalently bonded to the double-stranded nucleic acid by light irradiation;
a washing solution tank for holding a washing solution that removes the intercalator unreacted with the double-stranded nucleic acid;
an electrolyte tank for holding an electrolyte for detecting the intercalator covalently bonded to the double-stranded nucleic acid; and
a processing tank for forming a double-stranded nucleic acid by the nucleic acid probe immobilized onto the electrode and the gene sample, covalently bonding the double-stranded nucleic acid and the intercalator by light irradiation, washing the intercalator that is unreacted with the double-stranded nucleic acid by the washing solution, and detecting the intercalator covalently bonded to the double-stranded nucleic acid by electrochemical measurement, said processing tank being connected to the gene sample conformation unit, the intercalator tank, the washing solution tank, and the electrolyte tank.

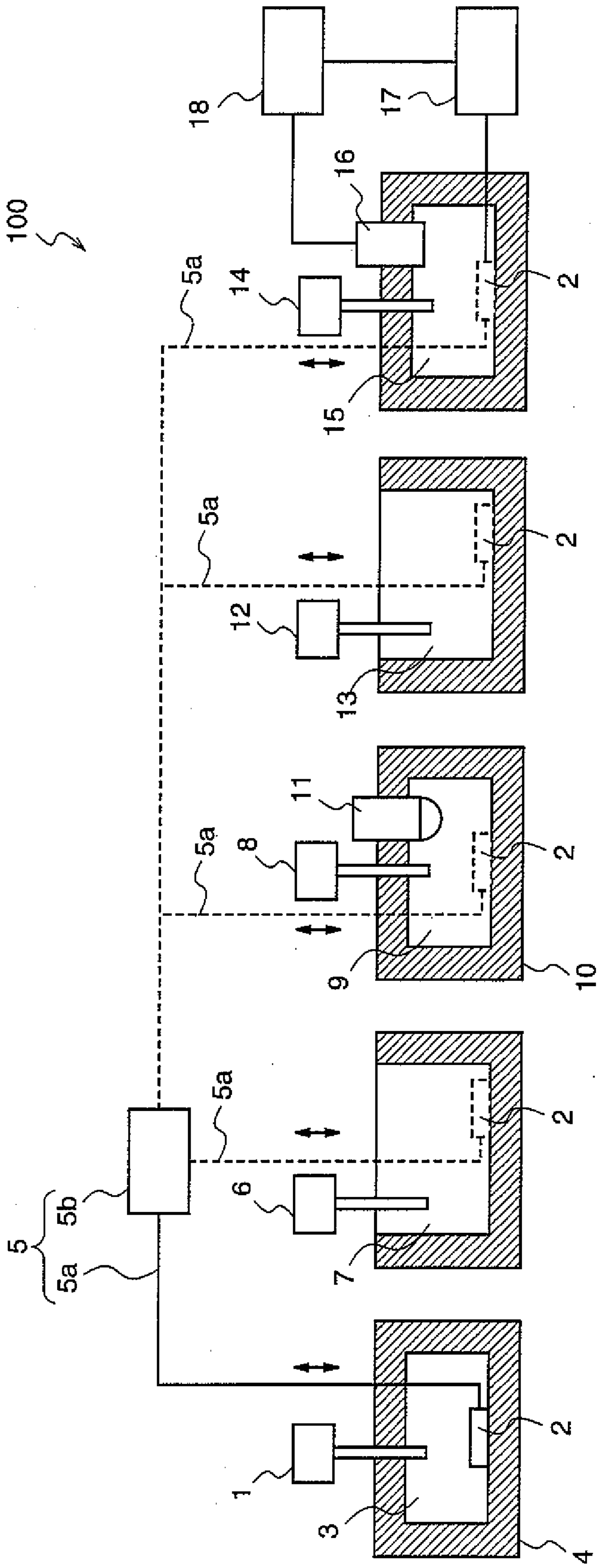


Fig.1

Fig.2

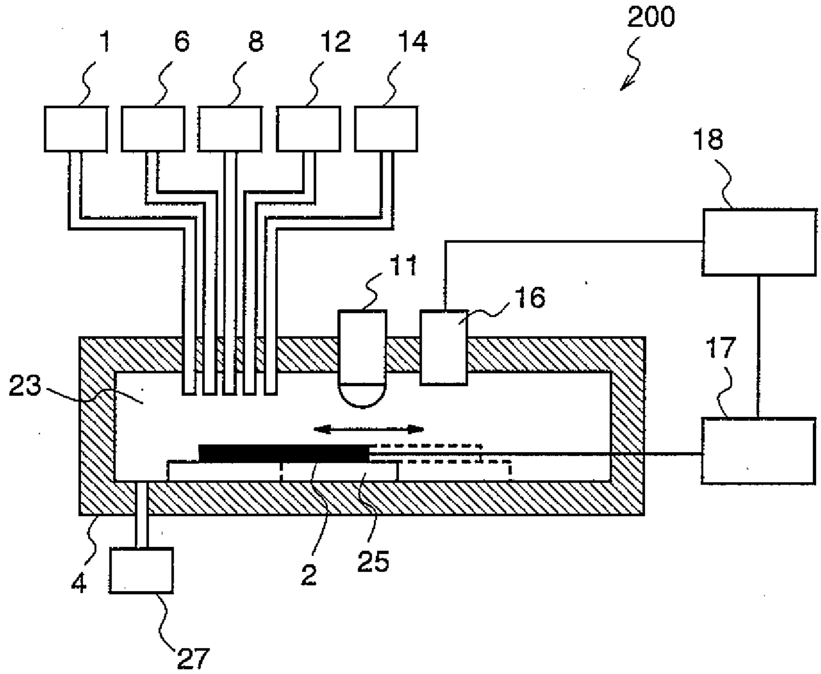
200
1
6
8
12
14
18
11
16
17
23
4
2
25
27

Fig.3

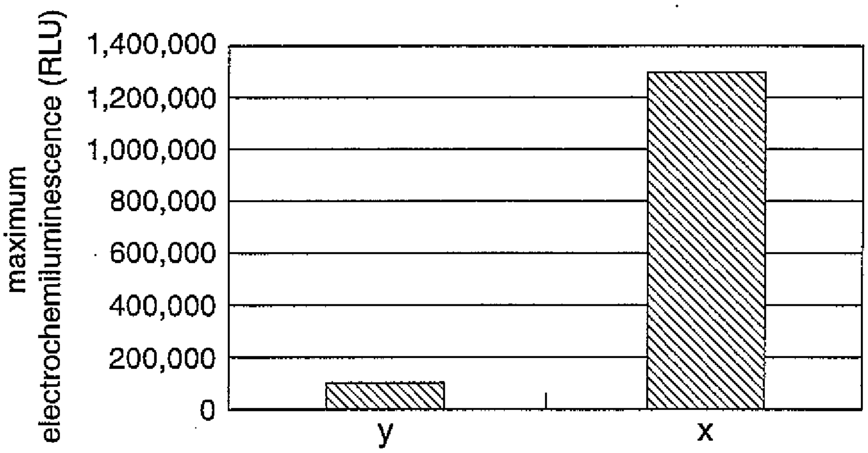
maximum electrochemiluminescence (RLU)
1,400,000
1,200,000
1,000,000
800,000
600,000
400,000
200,000
0
y
x

## INTERNATIONAL SEARCH REPORT

International application No.
PCT/JP2005/008374

A. CLASSIFICATION OF SUBJECT MATTER
Int.Cl$^7$ G01N33/53, C12Q1/68, G01N37/00//C12N15/09

According to International Patent Classification (IPC) or to both national classification and IPC

B. FIELDS SEARCHED

Minimum documentation searched (classification system followed by classification symbols)
Int.Cl$^7$ G01N33/53, C12Q1/68, G01N37/00//C12N15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| Y | JP 2002-524091 A (Nakkusuko Inc.), 06 August, 2002 (06.08.02), & WO 2000/014281 A & AU 9957793 A & EP 1105539 A & CN 1313908 A & KR 2001072846 A & MX 2001001894 A | 1-14 |
| Y | JP 7-506483 A (APPLIGEN S.A.), 20 July, 1995 (20.07.95), & WO 93/15223 A & FR 2686621 A & EP 581938 A & DE 69310278 E & US 5869236 A | 1-14 |
| Y | JP 2002-139491 A (Fuji Photo Film Co., Ltd.), 17 May, 2002 (17.05.02), & EP 1201767 A & US 2002/0177146 A | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 June, 2005 (14.06.05) | Date of mailing of the international search report<br>28 June, 2005 (28.06.05) |
| Name and mailing address of the ISA/<br>Japanese Patent Office<br>Facsimile No. | Authorized officer<br>Telephone No. |

Form PCT/ISA/210 (second sheet) (January 2004)

INTERNATIONAL SEARCH REPORT

International application No.
PCT/JP2005/008374

C (Continuation). DOCUMENTS CONSIDERED TO BE RELEVANT

| Category* | Citation of document, with indication, where appropriate, of the relevant passages | Relevant to claim No. |
|---|---|---|
| A | JP 61-500688 A (HRI RESEARCH, INC.), 10 April, 1986 (10.04.86), & WO 85/02628 A & EP 165313 A & US 4599303 A & CA 1236410 A & DE 3480487 G | 1-14 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2573443 A
- WO 3233851 A


### Non-patent literature cited in the description


- **M. MAEDA et al.** *Chem. Lett.,* 1994, 1805-1808
- **B.A. CONNOLLY.** *Nucleic Acids Res.,* 1985, vol. 13, 4484
- **K. M. MILLAN et al.** *Analytical Chemistry,* 1993, vol. 65, 2317-2323